# EUROPEAN PATENT APPLICATION

(11) **EP 4 541 820 A1**
(43) Date of publication of application: **23.04.2025**
(21) Application number: 23822958.7
(22) Date of filing: 05.06.2023
(51) Int. Cl.: C07K 16/38, A61K 39/395, C12N 15/13, A61P 7/04

(54) **MONOCLONAL ANTIBODY AGAINST TFPI AND USE THEREOF**

(30) Priority: 17.06.2022 CN 202210685961
(71) Applicant: Ampsource Biopharma Shanghai Inc., Shanghai 201318 (CN)
(72) Inventor: LI, Qiang, Shanghai 201318 (CN); WU, Cui, Shanghai 201318 (CN); DONG, Zhao, Shanghai 201318 (CN); ZHOU, Li, Shanghai 201318 (CN); ZHOU, Chi, Shanghai 201318 (CN)
(74) Representative: HGF
(86) International application number: PCT/CN2023/098242
(87) International publication number: WO 2023/241389

(57) **Abstract**

Antibodies or antigen-binding fragments thereof against human TFPI are provided, along with nucleic acid molecules encoding said antibodies, expression vectors and host cells for expressing said antibodies, and methods for producing said antibodies. Furthermore, pharmaceutical compositions containing the antibody or the antigen-binding fragment thereof are provided, as well as their use in the manufacture of a medicament for the prevention and/or treatment of coagulation disorders in patients with genetic or acquired coagulation factor deficiencies.

## Description

### TECHNICAL FIELD

This present invention pertains to the field of therapeutic monoclonal antibodies, in particular, it relates to an antibody or an antigen-binding fragment thereof against tissue factor pathway inhibitor (TFPI); and uses of the antibody in the preparation of a medicament for preventing or treating diseases or events associated with genetic or acquired coagulation factor deficiencies.

### BACKGROUND

Hemophilia A and B are inherited bleeding disorders characterized by impaired generation of active thrombin, prolonged coagulation time, and a lifelong tendency to bleed after minor trauma. Severe patients may experience spontaneous bleeding without apparent injury. Hemophilia A, accounting for over 80% of all hemophilia cases, is characterized by the absence or deficiency of coagulation factor VIII (FVIII), with a prevalence of 1/5,000 in males. Hemophilia B, characterized by the absence or deficiency of coagulation factor IX (FIX), affects approximately 1 in 30,000 newborns. Recurrent, spontaneous, and trauma-related bleeding are hallmarks of severe hemophilia, with typical bleeding sites being joints and muscles. Under minimal treatment, severe patients may experience up to 30-40 bleeds per year, leading to irreversible joint damage and secondary disability. The most commonly used treatment is factor replacement therapy, which involves supplementing FVIII or FIX in hemophilia patients. Based on current treatments, prophylactic therapy requires frequent infusions of FVIII or FIX, typically 2 to 4 times per week, to reduce spontaneous bleeding. However, while this treatment can effectively prevent most spontaneous bleeds, it cannot prevent trauma-induced bleeding. Furthermore, treatment efficacy is influenced by infusion frequency, dosage, and missed or delayed doses, which are in turn affected by factor elimination half-life, cost-effectiveness of the regimen, and patient acceptability. To overcome the limitations of factor replacement therapy, improve patient compliance, and reduce patient burden, new treatment methods need to be explored.

Tissue factor pathway inhibitor (TFPI) is a natural anticoagulant protein *in vivo* that controls the initiation phase of coagulation. It specifically inhibits the tissue factor-initiated coagulation pathway by binding to the TF/FVIIa complex, preventing the activation of factor X and subsequently inhibiting coagulation. When TFPI activity is neutralized by anti-TFPI antibodies, the coagulation process is restored or amplified. TFPI is a glycoprotein composed of 276 amino acid residues in its full length, containing three Kunitz domains (K1, K2, and K3), as well as two linker regions, an N-terminus rich in acidic amino acids, and a C-terminus rich in basic amino acids. Domains K1 and K2 are crucial for TFPI's anticoagulant activity, while K3 is not essential for this role but enhances TFPI's ability to inhibit coagulation. Recent studies have shown that K3 and the C-terminus can bind to heparin, membrane surface glycoproteins, and polysaccharides. Additionally, this region is associated with TFPI's anti-inflammatory effects. TFPI inhibits the coagulation pathway in two steps: first, TFPI binds to activated factor X through K2 and competitively inhibits its activity, a reversible process that is Ca²⁺-independent; second, the TFPI in the factor Xa/TFPI complex binds to the active site of factor VIIa in the FVIIa/TF complex through K1, thereby inhibiting the FVIIa/TF complex. Anti-TFPI antibodies act on the K2 domain of TFPI, competitively blocking the binding site of factor Xa to TFPI, elevating factor Xa levels, and also blocking TFPI's inhibitory effect on TF-FVIIa in the presence of factor Xa and Ca²⁺, resulting in increased factor Xa levels and thrombin generation, enhancing coagulation through a dual mechanism.

Anti-TFPI antibodies can be used in hemophilia patients with or without inhibitors to FVIII and FIX, and the antibodies themselves have a longer half-life, reducing the frequency of dosing. Hemophilia treatment antibodies targeting different Kunitz domains are currently being studied in clinical trials. For example, humanized monoclonal antibody Concizumab developed by Novo Nordisk has entered Phase III clinical trials, monoclonal antibody Marstacimab developed by Pfizer has also entered Phase III clinical trials, and BAY-1093884 developed by Bayer is in Phase II clinical trials. Currently, there are no approved antibody drugs targeting TFPI on the market.

### SUMMARY

The present invention provides an antibody that specifically binding to human TFPI, a nucleic acid encoding said antibody, a vector containing said nucleic acid, a host cell containing said vector, a method for producing said antibody, and a pharmaceutical composition for preventing or treating coagulation disorders in patients with genetic or acquired coagulation factor deficiencies, wherein the pharmaceutical composition contains the antibody as an active ingredient and have ability of inhibiting TFPI to activate the coagulation pathway. The anti-human TFPI antibody disclosed in this invention can be used for treating or preventing hemophilia.

In the first aspect of the present invention, the antibody or the antigen-binding fragment thereof provided herein can specifically binding to TFPI, wherein the heavy chain variable region (VH) of the antibody or antigen-binding fragment thereof contains at least one, two, or three complementarity determining regions (CDRs) selected from the group consisting of:
(1) HCDR1 having a sequence as shown in SEQ ID NO: 1 or 7, or a sequence having one or several amino acid substitutions, deletions, or additions (such as 1, 2, or 3 substitutions, deletions, or additions) relative to any of the sequences;
(2) HCDR2 having a sequence as shown in SEQ ID NO: 2, 8, 12, or 13, or a sequence having one or several amino acid substitutions, deletions, or additions (such as 1, 2, or 3 substitutions, deletions, or additions) relative to any of the sequences; and
(3) HCDR3 having a sequence as shown in SEQ ID NO: 3 or 9, or a sequence having one or several amino acid substitutions, deletions, or additions (such as 1, 2, or 3 substitutions, deletions, or additions) relative to any of the sequences;
and/or, the light chain variable region (VL) of said antibody or antigen-binding fragment thereof contains at least one, two, or three CDRs selected from the group consisting of:
(4) LCDR1 having a sequence as shown in SEQ ID NO: 4 or 10, or a sequence having one or several amino acid substitutions, deletions, or additions (such as 1, 2, or 3 substitutions, deletions, or additions) relative to any of the sequences;
(5) LCDR2 having a sequence as shown in SEQ ID NO: 5 or 11 or Gly-Thr-Ser (SEQ ID NO: 11), or a sequence having one or several amino acid substitutions, deletions, or additions (such as 1, 2, or 3 substitutions, deletions, or additions) relative to any of the sequences; and
(6) LCDR3 having a sequence as shown in SEQ ID NO: 6, or a sequence having one or several amino acid substitutions, deletions, or additions (such as 1, 2, or 3 substitutions, deletions, or additions) relative to any of the sequences.

In certain preferred embodiments, the substitutions mentioned in any of (1)-(6) are conservative substitutions.

In certain preferred embodiments, the HCDR1, HCDR2, and HCDR3 contained in the heavy chain variable region, and/or the LCDR1, LCDR2, and LCDR3 contained in the light chain variable region, are defined by either the Kabat or IMGT numbering system. Table 2 in Example 3 exemplarily provides the CDR amino acid sequences of murine antibodies defined according to the Kabat or IMGT numbering system.

In certain preferred embodiments, the antibody or the antigen-binding fragment thereof comprises three VH variable region CDRs and three VL variable region CDRs, selected from the following group:
(1) The HCDR1, HCDR2, HCDR3, LCDR1, LCDR2, and LCDR3 have the sequences as shown in SEQ ID NOs: 1, 2, 3, 4, 5, or 6, respectively, or sequences with one or several amino acid substitutions, deletions, or additions (such as 1, 2, or 3 substitutions, deletions, or additions) relative to any of the sequences;
(2) The HCDR1, HCDR2, HCDR3, LCDR1, LCDR2, and LCDR3 have the sequences as shown in SEQ ID NOs: 7, 8, 9, 10, Gly-Thr-Ser (SEQ ID NO: 11), or 6, respectively, or sequences with one or several amino acid substitutions, deletions, or additions (such as 1, 2, or 3 substitutions, deletions, or additions) relative to any of the sequences;
(3) The HCDR1, HCDR2, HCDR3, LCDR1, LCDR2, and LCDR3 have the sequences as shown in SEQ ID NOs: 1, 12, 3, 4, 5, or 6, respectively, or sequences with one or several amino acid substitutions, deletions, or additions (such as 1, 2, or 3 substitutions, deletions, or additions) relative to any of the sequences;
(4) The HCDR1, HCDR2, HCDR3, LCDR1, LCDR2, and LCDR3 have the sequences as shown in SEQ ID NOs: 7, 13, 9, 10, Gly-Thr-Ser (SEQ ID NO: 11), or 6, respectively, or sequences with one or several amino acid substitutions, deletions, or additions (such as 1, 2, or 3 substitutions, deletions, or additions) relative to any of the sequences.

In certain embodiments, the antibody or the antigen-binding fragment thereof is murine-derived or chimeric, with its heavy chain variable region containing a heavy chain FR region derived from murine IgG1, IgG2, IgG3, or a variant thereof; and its light chain variable region containing a light chain FR region derived from murine κ or λ chain, or a variant thereof. Table 3 in Example 3 provides the amino acid sequence numbering for the variable regions of preferred murine-derived antibodies.

In certain preferred embodiments, the murine-derived or chimeric antibody or the antigen-binding fragment thereof comprises the following VH and VL sequences:
the VH domain comprises an amino acid sequence as shown in SEQ ID NO: 14, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 14; and the VL domain comprises an amino acid sequence as shown in SEQ ID NO: 15, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 15.

In certain embodiments, the antibody or the antigen-binding fragment thereof is humanized. Example 3 outlines the basic process of the humanization strategy, with Table 2 exemplarily providing the CDR amino acid sequences of preferred humanized antibodies defined according to the Kabat or IMGT numbering system, and Table 3 providing the amino acid sequence numbering for the variable regions of preferred humanized antibodies.

In certain preferred embodiments, the humanized antibody or its antigen-binding fragment comprises the following VH and VL sequences: the VH domain comprises an amino acid sequence as shown in SEQ ID NO: 16, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 16; and the VL domain comprises an amino acid sequence as shown in SEQ ID NO: 17, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 17.

In certain embodiments, the antibody comprises a heavy chain constant region and a light chain constant region derived from human immunoglobulins.

Preferably, the antibody includes an amino acid sequence of the human κ light chain constant region (as shown in SEQ ID NO: 18).

More preferably, the antibody contains a heavy chain constant region selected from human IgG1, IgG2, IgG3, IgG4, IgM, IgA1, IgA2, IgD, and IgE; even more preferably, it contains a heavy chain constant region selected from human IgG1, IgG2, and IgG4; and the heavy chain constant region has a natural sequence or a sequence with one or more amino acid substitutions, deletions, or additions compared to the natural sequence it is derived from. For example, in one embodiment, the humanized antibody molecule contains the heavy chain constant region of wild-type human IgG1 (with an amino acid sequence as shown in SEQ ID NO: 19). In another embodiment, the humanized antibody molecule includes the heavy chain constant region of human IgG1 with M252Y, S254T, T256E, and M428L mutations according to EU numbering (with an amino acid sequence as shown in SEQ ID NO: 20). In another embodiment, the humanized antibody molecule contains the heavy chain constant region of wild-type human IgG2 (with an amino acid sequence as shown in SEQ ID NO: 21). In another embodiment, the humanized antibody molecule includes human IgG4 with a mutation at position 228 (such as S to P) according to EU numbering (with an amino acid sequence as shown in SEQ ID NO: 22).

In an exemplary embodiment, the humanized antibody molecule has a heavy chain amino acid sequence as shown in SEQ ID NO: 23 and a light chain amino acid sequence as shown in SEQ ID NO: 24.

In any of the aforementioned embodiments, the antibody or the antigen-binding fragment thereof is capable of binding to TFPI with a KD of 10 nM or lower, more preferably with a KD of 1 nM or lower; even more preferably, with a KD of 100 pM or lower; and most preferably, with a KD of 10 pM or lower.

In a second aspect of the invention, a polynucleotide seqeunce encoding the antibody or the antigen binding fragment thereof is provided.

In a third aspect of the present invention, a vector comprising the polynucleotide sequence is provided.

In a fourth aspect of the invention, a host cell containing the vector is provided; the host cells comprise prokaryotic cells, yeast cells, or mammalian cells, such as CHO cells, NS0 cells, or other mammalian cells, preferably CHO cells.

In a fifth aspect of the invention, a pharmaceutical composition comprising the antibody or the antigen-binding fragment thereof, and a pharmaceutically acceptable excipient, carrier, or diluent.

In a sixth aspect of the invention, further provides a method for preparing the antibody or the antigen-binding fragment thereof, which includes: (a) obtaining a gene encoding the antibody or the antigen-binding fragment thereof and constructing an expression vector for the antibody or the antigen-binding fragment thereof; (b) transfecting the expression vector into a host cell using genetic engineering methods; (c) culturing the host cells under conditions that allow for the production of the antibody or the antigen-binding fragment thereof; (d) isolating and purifying the produced antibody or its antigen-binding fragment.

In step (a), the expression vector is selected from one or more of plasmids, bacteria, and viruses, preferably pcDNA3.1.

In step (b), the constructed vector is transfected into the host cells using genetic engineering methods, wherein the host cells include prokaryotic cells, yeast cells, or mammalian cells such as CHO cells, NS0 cells, or other mammalian cells, preferably CHO cells.

In step (d), the antibody or the antigen-binding fragment thereof is isolated and purified using conventional immunoglobulin purification methods, including Protein A affinity chromatography and ion exchange methods.

In a seventh aspect of the invention, provides the use of the antibody or the antigen-binding fragment thereof in the preparation of a medicament for preventing or treating diseases or events associated with genetic or acquired coagulation factor deficiencies. For example, the antibody or the antigen-binding fragment thereof provided herein can be used to block the interaction between TFPI and FXa, or to prevent TFPI-dependent inhibition of TF/FVIIa activity. Additionally, the human monoclonal antibody can be used to restore TF/FVIIa-driven FXa generation, thereby bypassing the insufficiency of FVIII or FIX-dependent FXa amplification. Furthermore, the diseases include hemophilia A and hemophilia B, as well as spontaneous bleeding events, traumatic bleeding events, or bleeding events occurring during prophylactic treatment, perioperative management, or surgical treatment in hemophilia patients.

In other aspects of the invention, provides a method for preventing or treating diseases or events associated with genetic or acquired coagulation factor deficiencies, which includes administering an effective amount of the antibody or the antigen-binding fragment thereof, or the pharmaceutical composition.

### Abbreviations and Definitions of Terms

The following abbreviations are used herein:
CDR - Complementarity-Determining Region, the immunoglobulin variable region that binds antigens, defined by the Kabat, IMGT, Chothia, or AbM numbering systems (see terms "hypervariable region," "CDR region," or "complementarity-determining region").
EC₅₀ - Concentration required to achieve 50% efficacy or binding
ELISA - Enzyme-Linked Immunosorbent Assay
FR - Framework Region of an antibody, the immunoglobulin variable region excluding the CDRs
HRP - Horseradish Peroxidase
IC₅₀ - Concentration required to achieve 50% inhibition
IgG - Immunoglobulin G
Kabat - An immunoglobulin amino acid sequence alignment and numbering system advocated by Elvin A Kabat.
PCR - Polymerase Chain Reaction
V Region - The segment of the IgG chain that varies in sequence among different antibodies. It extends to residue 109 of the Kabat numbering in the light chain and residue 113 in the heavy chain.
K_{D} - Equilibrium dissociation constant
kₐ - Association rate constant
k_{d} - Dissociation rate constant

In this present invention, unless otherwise specified, the scientific and technical terms used herein have the meanings generally understood by those skilled in the art. Additionally, the operational steps used herein, such as cell culture, biochemistry, nucleic acid chemistry, and immunological laboratory procedures, are all conventional steps widely used in the art. Meanwhile, for a better understanding of this invention, definitions and explanations of relevant terms are provided below.

The term "Tissue Factor Pathway Inhibitor" or "TFPI" refers to any variant, isoform, and species homolog of human TFPI naturally expressed by cells.

Eu in the term "EU Numbering System or Scheme" refers to the first human immunoglobulin IgG1 separated and purified by Gerald M Edelman et al. at the end of the 1960s (1968-1969), where the amino acid sequence of IgG1 was determined and numbered (Edelman GM et al, 1969, Proc Natl Acad USA, 63: 78-85). A heavy chain constant region of another immunoglobulin is aligned with the amino acid sequence of Eu, and the corresponding amino acid positions are Eu numbers. The Eu numbering system is mainly directed to a heavy chain constant region of an immunoglobulin, including CH1, CH2, CH3 and a hinge region.

The term "Kabat Numbering System or Scheme" refers to a standardized numbering scheme of variable regions of human immunoglobulins firstly proposed by Kabat et al. in 1979 (Kabat EA, Wu TT, Bilofsky H, Sequences of Immunoglobulin Chains: Tabulation and Analysis of Amino Acid Sequences of Precursors, V-regions, C-regions, J-Chain and β2-Microglobulins. 1979. Department of Health, Education, and Welfare, Public Health Service, National Institutes of Health). In the book "Sequences of Proteins of Immunological Interest" (Kabat EA, Wu TT, Perry HM, Gottesman KS, Foeller C. 1991. 5th edition. Bethesda, MD: US Department of Health and Human Services, National Institutes for Health), Kabat et al. aligned and numbered the amino acid sequences of antibody light and heavy chains. They found that these analyzed sequences exhibited variable lengths, with default and inserted amino acids or amino acid fragments only appearing at specific positions. Interestingly, insertion points are mostly located within CDRs, but they may also appear at certain positions in the framework regions. In the Kabat numbering scheme, the light chain variable region is numbered up to position 109, and the heavy chain variable region is numbered up to position 113. Inserted amino acids in both light and heavy chains are identified and annotated with letters (such as 27a, 27b...). All Lambda light chains do not contain the residue at position 10, and Lambda and Kappa light chains are encoded by two different genes located on different chromosomes. Lambda and Kappa light chains can be distinguished by differences in their constant region amino acid sequences. Unlike the EU numbering system, which focuses only on the constant region of the heavy chain, the Kabat numbering system covers the full-length immunoglobulin sequence, including the variable and constant regions of both immunoglobulin light and heavy chains.

The term "antibody" generally refers to proteinaceous binding molecules with immunoglobulin-like functions. Typical examples of antibodies are immunoglobulins, as well as their derivatives or functional fragments, as long as they exhibit the desired binding specificity. Techniques for preparing the antibody are well-known in the art. The "antibody" includes different classes of natural immunoglobulins (such as IgA, IgG, IgM, IgD, and IgE) and subclasses (such as IgG1, IgG2, IgA1, IgA2, etc.). The "antibody" further includes non-natural immunoglobulins which include, for example, a single-chain antibody, a chimeric antibody (for example, humanized murine antibody) and a heterologous conjugated antibody (for example, bispecific antibody) as well as antigen-binding fragments thereof (for example, Fab', F(ab')2, Fab, Fv and rIgG). See also, for example, Pierce Catalog and Handbook, 1994-1995 (Pierce Chemical Co., Rockford, IL); Kuby J, Immunology, 3rd Ed., WH Freeman & Co., New York, 1997. The antibody can bind to one antigen, referred to as "monospecific"; or to two different antigens, referred to as "bispecific"; or to more than one different antigen, referred to as "multispecific". The antibody may be monovalent, bivalent, or multivalent, meaning that the antibody may bind to one, two, or multiple antigen molecules at one time. The antibody "monovalently" binds to a particular protein, that is, one molecule of the antibody binds only to one molecule of the protein, but the antibody may also bind to different proteins. When the antibody binds only to one molecule of each of two different proteins, the antibody binds "monovalently" to each protein, and the antibody is "bispecific" and binds "monovalently" to each of the two different proteins. The antibody may be "monomeric," that is, the antibody contains a single polypeptide chain. The antibody may contain multiple polypeptide chains ("multimeric ") or may contain two ("dimeric"), three ("trimeric"), or four ("tetrameric") polypeptide chains. If the antibody is multimeric, the antibody may be a homomultimer, that is, the antibody contains more than one molecule of only one type of polypeptide chain, including a homodimer, a homotrimer, or a homotetramer. Alternatively, the multimeric antibody may be a heteromultimer, that is, the antibody contains more than one type of polypeptide chains that are different, including a heterodimer, a heterotrimer or a heterotetramer.

The term "monoclonal antibody (mAb)" refers to an antibody obtained from a population of substantially homogeneous antibodies, that is, individual antibodies comprised in the population are identical except for possible naturally occurring mutations that present in minor amounts. Therefore, the modifier "monoclonal" means that the property of the antibody is not a mixture of discrete antibodies. The monoclonal antibody is produced by methods known to those skilled in the art, such as the preparation of heterozygous antibody-producing cells through the fusion of myeloma cells and immune spleen cells. The monoclonal antibody can be synthesized by culturing hybridomas without being contaminated by any other immunoglobulins. The monoclonal antibody may be obtained through recombination technology, phage display technology, synthesis technology or other existing technologies.

The term "intact antibody" refers to an antibody composed of two antibody heavy chains and two antibody light chains. An "intact antibody heavy chain" consists of an antibody heavy chain variable domain (VH), an antibody constant heavy chain domain 1 (CH1), an antibody hinge region (HR), an antibody heavy chain constant domain 2 (CH2), and an antibody heavy chain constant domain 3 (CH3) from N-terminus to C-terminus, abbreviated as VH-CH1-HR-CH2-CH3; and optionally, in the case of IgE subclass antibodies, it also includes an antibody heavy chain constant domain 4 (CH4). Preferably, the "intact antibody heavy chain" is a polypeptide consisting of VH, CH1, HR, CH2, and CH3 from the N-terminus to the C-terminus. An "intact antibody light chain" is a polypeptide consisting of an antibody light chain variable domain (VL) and an antibody light chain constant domain (CL) from N-terminus to C-terminus, abbreviated as VL-CL. The antibody light chain constant domain (CL) may be κ (kappa) or λ (lambda). The Intact antibody chains are linked together by interpeptide disulfide bonds between the CL domain and the CH1 domain (i.e., between the light chain and the heavy chain) and by interpeptide disulfide bonds within the hinge region of the intact antibody heavy chains. Typical examples of intact antibody are natural antibodies such as IgG (for example, IgG1 and IgG2), IgM, IgA, IgD, and IgE.

The term "antibody fragment" or "antigen-binding fragment" refers to an antigen-binding fragment of an antibody that retains a specific binding ability to an antigen as well as antibody analogs and generally includes at least part of an antigen-binding region or a variable region of a parental antibody. The antibody fragment retains at least part of the binding specificity of the parental antibody. Generally, when activity is represented in moles (KD), the antibody fragment retains at least 10% of parental binding activity. Preferably, the antibody fragment retains at least 20%, 50%, 70%, 80%, 90%, 95% or 100% of the binding affinity of the parental antibody to a target. Antibody fragments include, but are not limited to, Fab fragments, Fab' fragments, F(ab')2 fragments, Fv fragments, Fd fragments, complementarity-determining region (CDR) fragments, disulfide-stabilized variable fragments (dsFv); linear antibodies, single-chain antibodies (for example, scFv monoclonal antibodies), unibodies (technology from Genmab), bivalent single-chain antibodies, single-chain phage antibodies, single domain antibodies (for example, VH domain antibodies), domain antibodies (technology from Domantis), nanobodies (technology from Ablynx); multi-specific antibodies formed from antibody fragments (for example, tribodies and tetrabodies); and engineered antibodies such as chimeric antibodies (for example, humanized murine antibodies) and heteroconjugate antibodies. These antibody fragments are obtained using any conventional technologies known to those skilled in the art, and the utility of these fragments is screened by the same method as the intact antibody.

The term "single-chain Fv antibody" (or "scFv antibody") refers to an antibody fragment comprising VH and VL domains of an antibody and is a fusion protein of a heavy chain variable region (VH) and a light chain variable region (VL) connected with a linker. The linker enables the two domains to be cross-linked to form an antigen-binding site, and the sequence of the linker generally consists of a flexible peptide, such as, but not limited to, G2(GGGGS)3. The size of scFv is generally 1/6 of an intact antibody. The single-chain antibody is preferably an amino acid chain sequence encoded by a nucleotide chain. For the review of scFv, reference may be made to Pluckthun A, 1994. Antibodies from Escherichia coli, in the Pharmacology of Monoclonal Antibodies, Vol 113, Rosenberg M and Moore GP (EDs.), Springer-Verlag, New York, pp 269-315. Reference may also be made to International Patent Application Publication No. WO 88/01649 and U.S. Patent Nos. 4946778 and 5260203.

The term "VL domain" refers to the amino-terminal variable region domain of the immunoglobulin light chain.

The term "VH domain" refers to the amino-terminal variable region domain of the immunoglobulin heavy chain.

The term "hinge region" encompasses the portion of the heavy chain molecule that connects the CH1 domain to the CH2 domain. This hinge region comprises approximately 25 residues and is flexible, allowing the two N-terminal antigen-binding regions to move independently. The hinge region can be subdivided into three distinct domains: the upper, middle, and lower hinge domains (Roux KH et al., 1998, J. Immunol., 161:4083-4090).

The term "Fv region" encompasses the variable regions from both the heavy and light chains but lacks the constant regions, and is the minimum fragment that contains the complete antigen recognition and binding site.

The term "heavy chain constant region" includes an amino acid sequence from an immunoglobulin heavy chain. A polypeptide comprising the heavy chain constant region includes at least one of: a CH1 domain, a hinge domain (for example, an upper hinge region, an intermediate hinge region and/or a lower hinge region), a CH2 domain, a CH3 domain or a variant or fragment thereof. For example, an antigen-binding polypeptide used in the present application may include a polypeptide chain having a CH1 domain; a polypeptide having a CH1 domain, at least part of a hinge domain and a CH2 domain; a polypeptide chain having a CH1 domain and a CH3 domain; a polypeptide chain having a CH1 domain, at least part of a hinge domain and a CH3 domain; or a polypeptide chain having a CH1 domain, at least part of a hinge domain, a CH2 domain and a CH3 domain. In another embodiment, the polypeptide of the present application includes a polypeptide chain having a CH3 domain. In addition, the antibody used in the present application may lack at least part of a CH2 domain (for example, all or part of the CH2 domain). As described above, it is appreciated by those of ordinary skill in the art that heavy chain constant regions may be modified such that they differ in amino acid sequence from naturally occurring immunoglobulin molecules.

The term "light chain constant region" encompasses amino acid sequences derived from the antibody light chain. Preferably, the light chain constant region includes at least one of the constant kappa domain and the constant lambda domain.

The term "Fc region" or "Fc fragment" refers to a C-terminal region of an immunoglobulin heavy chain, which includes at least part of a hinge region, a CH2 domain and a CH3 domain. It mediates the binding of immunoglobulins to host tissues or factors, including the binding of immunoglobulins to Fc receptors located on various cells (for example, effector cells) of an immune system or the binding of immunoglobulins to the first component (C1q) of a classical complement system. The Fc region includes a native sequence Fc region and a variant Fc region.

Generally, a human IgG heavy chain Fc region is a segment from an amino acid residue at position Cys226 or Pro230 of the human IgG heavy chain Fc region to the carboxy terminus, but its boundary may vary. The C-terminal lysine of the Fc region (residue 447, according to the EU numbering system) may or may not be present. Fc may also refer to this region in isolation or in the case of a protein polypeptide comprising Fc, for example, a "binding protein comprising the Fc region", which is also referred to as "Fc fusion protein" (for example, antibody or immunoadhesin). The native sequence Fc region of the antibody of the present application includes mammalian (for example, human) IgG1, IgG2 (IgG2A or IgG2B), IgG3 and IgG4. In some embodiments, there is a single amino acid substitution, insertion and/or deletion of about 10 amino acids per 100 amino acids in the amino acid sequences of two Fc polypeptide chains relative to the amino acid sequence of the mammalian Fc polypeptide. In some embodiments, the amino acid difference in the Fc region may be Fc changes that extend a half-life, changes that increase FcRn binding, changes that enhance Fcγ receptor (FcγR) binding and/or changes that enhance ADCC, ADCP and/or CDC.

As used herein, the term "immune response" refers to the action of immune cells (such as lymphocytes, antigen-presenting cells, phagocytes, or granulocytes) and soluble macromolecules produced by immune cells or the liver (including antibodies, cytokines, and complements) that results in selective damage, destruction, or elimination from the human body of invasive pathogens, cells or tissues infected by pathogens, cancer cells, or normal human cells or tissues in cases of autoimmunity or pathological inflammation. In the present invention, the term "antigen-specific T-cell response" refers to an immune response produced by T-cells that arises when the T-cells are stimulated by their specific antigen. Non-limiting examples of the reactions produced by T-cells upon antigen-specific stimulation include proliferation of the T-cells and production of cytokines (such as IL-2).

As used herein, the term "Antibody-Dependent Cell-Mediated Cytotoxicity (ADCC)" refers to a form of cytotoxicity whereby IgG binds to Fc receptors (FcR) present on cytotoxic cells (such as Natural Killer (NK) cells, neutrophils, or macrophages), causing these cytotoxic effector cells to specifically bind to target cells with attached antigen, and then killing the target cells by secreting cytotoxins. Methods for detecting the ADCC activity of an antibody are known in the art, for example, by assessing the binding activity between a to-be-tested antibody and Fc receptors (for example,CD16a).

As used herein, the term "Complement-Dependent Cytotoxicity (CDC)" refers to a form of cytotoxicity that activates the complement cascade by enabling the binding of complement component C1q to the Fc region of an antibody. Methods for detecting the CDC activity of an antibody are known in the art, for example, by assessing the binding activity between a to-be-tested antibody and Fc receptors (such as C1q).

In the IgG, IgA, and IgD antibody isotypes, the Fc region comprises the CH2 and CH3 constant domains of each of the two heavy chains of the antibody; whereas the IgM and IgE Fc regions encompass three heavy chain constant domains (CH2-4 domains) in each polypeptide chain.

The term "humanized antibody" refers to a non-human antibody that has been genetically engineered with its amino acid sequence modified to increase homology with the sequence of a human antibody. Most or all of the amino acids outside the CDR regions of the non-human antibody, for instance, a mouse antibody, are replaced with corresponding amino acids from human immunoglobulins, while most or all of the amino acids within one or more CDR regions remain unchanged. Additions, deletions, insertions, substitutions, or modifications of amino acids are permissible as long as they do not abolish the antibody's ability to bind to a specific antigen. "Humanized" antibodies retain antigen specificity similar to the original antibody. The source of CDRs is not particularly limited and can originate from any animal. For example, CDR regions derived from mouse antibodies, rat antibodies, rabbit antibodies, or non-human primate (such as cynomolgus monkey) antibodies can be utilized. The framework region can be obtained by searching the IMGT antibody germline database (http://www.imgt.org/3Dstructure-DB/cgi/DomainGapAlign.cgi) to identify human germline antibody sequences with high homology to the non-human antibody being engineered, and typically, a human germline antibody sequence with a high degree of homology is selected as the framework region for the humanized antibody.

The term "hypervariable region," "CDR region," or "complementarity-determining region" refers to the antibody amino acid residues responsible for antigen binding, which are discontinuous amino acid sequences. The CDR region sequences can be defined by the Kabat, Chothia, IMGT (Lefranc et al., 2003, Dev Comparat Immunol, 27:55-77) methods or any other well-known CDR region sequence determination methods in the field to identify amino acid residues within the variable region. For instance, hypervariable regions encompass amino acid residues from the "complementarity-determining regions" or "CDRs" defined by sequence alignment (Kabat numbering system), such as residues at positions 24-34 (LCDR1), 50-56 (LCDR2), and 89-97 (LCDR3) in the light chain variable domain and residues at positions 31-35 (HCDR1), 50-65 (HCDR2), and 95-102 (HCDR3) in the heavy chain variable domain, as described in Kabat et al., 1991, Sequences of Proteins of Immunological Interest, 5th Edition, Public Health Service, National Institutes of Health, Bethesda, Md.; and/or residues from the "hypervariable loops" (HVLs) defined by structure (Chothia numbering system), such as residues at positions 26-32 (LCDR1), 50-52 (LCDR2), and 91-96 (LCDR3) in the light chain variable domain and residues at positions 26-32 (HCDR1), 53-55 (HCDR2), and 96-101 (HCDR3) in the heavy chain variable domain, as described in Chothia C and Lesk AM, 1987, J Mol Biol, 196:901-917; Chothia C et al., 1989, Nature, 342:878-883. "Framework" residues or "FR" residues are the variable domain residues excluding the hypervariable region residues defined herein. In certain embodiments, the CDRs contained in the antibodies or their antigen-binding fragments of the invention are preferably determined by the Kabat or IMGT numbering system. Those skilled in the art can unequivocally assign each numbering system to any variable domain sequence without relying on any experimental data beyond the sequence itself. For example, the Kabat residue numbering for a given antibody can be determined by aligning the antibody sequence with each "standard" numbered sequence in the homologous region. Based on the sequence numbering schemes provided herein, determining the numbering of any variable region sequence in the sequence listing is entirely within the routine skill of those in the art.

The term "isolated" in reference to nucleic acids (such as DNA or RNA), as used herein, refers to molecules isolated from other DNA or RNA present as macromolecules of natural origin. The term "isolated" used herein also refers to nucleic acids or peptides that are essentially free of cellular material, viral material, or culture medium when produced by recombinant DNA technology, or essentially free of chemical precursors or other chemicals when prepared by chemical synthesis. In addition, "isolated nucleic acids" refer to nucleic acid fragments that are not naturally generated as fragments and are not present in the natural state. The term "isolated" used herein also refers to cells or polypeptides isolated from other cellular proteins or tissues. Isolated polypeptides include both purified and recombinant polypeptides.

The term "cross-reaction" refers to the ability of the antibody described herein to bind to antigens from different species. Cross-reactivity may be measured by detecting specific reactivity with purified antigens in binding assays (for example, SPR and ELISA) or by detecting the binding to cells physiologically expressing the antigen or by detecting the functionally interacting with such cells in other ways. Examples of assays known in the art for determining the binding affinity include surface plasmon resonance (for example, Biacore) or similar techniques (for example, Kinexa or Octet).

The terms "immunobinding" and "immunobinding properties" refer to a non-covalent interaction that occurs between an immunoglobulin molecule and an antigen for which the immunoglobulin is specific. The strength or affinity of the immunobinding interaction can be expressed in terms of the equilibrium dissociation constant (KD) of the interaction, where a smaller KD value indicates a higher affinity. The immunobinding properties of a selected polypeptide can be determined using methods well-known in the art. One method of determination involves measuring the rates of formation and dissociation of antigen/antibody complexes. Both the "association rate constant" (Ka or Kon) and the "dissociation rate constant" (Kd or Koff) can be calculated from the concentrations and the actual rates of association and dissociation (see Malmqvist M, 1993, Nature, 361:186-187). The ratio kd/ka is equal to the equilibrium dissociation constant KD (see Davies DR et al, 1990, Annual Rev Biochem, 59:439-473). Any effective method can be used to measure the values of KD, ka, and kd.

The term "host cell" refers to a cell in which a vector can proliferate and its DNA can be expressed, and this cell can be either prokaryotic or eukaryotic. This term also includes any offspring of the test host cell. It should be understood that not all offspring are identical to the parent cell, as mutations may occur during replication, and such offspring are included. The host cells include prokaryotic cells, yeast cells or mammalian cells such as CHO cells, NS0 cells or other mammalian cells.

The term "identity" refers to the matching of sequences between two polypeptides or between two nucleic acids. When a certain position in each of two sequences for comparison is occupied by the same base group or amino acid monomer subunit (for example, a certain position in each of two DNA molecules is occupied by adenine or a certain position in each of two polypeptides is occupied by lysine), then the molecules are identical at the position. The "percentage identity" between two sequences is a function of the number of matching positions of the two sequences divided by the number of positions to be compared and then multiplied by 100. For example, if 6 positions of 10 positions in two sequences are matched, the two sequences have 60% identity. For example, DNA sequences CTGACT and CAGGTT have a total identity of 50% (three positions of a total of six positions are matched). Generally, the comparison is made when two sequences are aligned to produce maximum identity. Such alignment may be conveniently implemented through a computer program, such as an Align program (DNAstar, Inc.), by the method described by Needleman and Wunsch (Needleman SB and Wunsch CD, 1970, J Mol Biol, 48: 443-453).

The term "mutated", "mutant" or "mutation" refers to the substitution, deletion or insertion of one or more nucleotides or amino acids relative to natural nucleic acids or polypeptides (i.e., reference sequences that may be used for defining wild types).

The term "conservative modification" is intended to mean that an amino acid modification does not significantly affect or alter the binding characteristics of an antibody containing that amino acid sequence. Such conservative modifications include substitutions, additions, and deletions of amino acids. Modifications can be introduced into the antibodies of the present invention through standard techniques known in the art, such as site-directed mutagenesis and PCR-mediated mutagenesis. A conservative amino acid substitution refer to the substitution of an amino acid residue with another amino acid residue that has a similar side chain. Families of amino acid residues with similar side chains have been described in detail in the art. These families include amino acids with basic side chains (such as lysine, arginine, histidine), amino acids with acidic side chains (such as aspartic acid, glutamic acid), amino acids with uncharged polar side chains (such as glycine, asparagine, glutamine, serine, threonine, tyrosine, cysteine, tryptophan), amino acids with nonpolar side chains (such as alanine, valine, leucine, isoleucine, proline, phenylalanine, methionine), amino acids with β-branched side chains (such as threonine, valine, isoleucine), and amino acids with aromatic side chains (such as tyrosine, phenylalanine, tryptophan, histidine). Therefore, one or more amino acid residues in the CDR regions of the antibody of the present invention may be substituted with other amino acid residues from the same side chain family.

The antibody of the present invention, or the nucleic acids or polynucleotides encoding the antibody, may be applied to the preparation of a pharmaceutical compositions or a sterile compositions, for instance, by mixing the antibody with pharmaceutically acceptable carriers, excipients, or stabilizers. The pharmaceutical compositions may include one or a combination (for example, two or more different) of the antibodies of the present invention. For example, the pharmaceutical compositions of the present invention may contain a combination of antibodies or antibody fragments (or immunoconjugates) with complementary activities that bind to different epitopes on the target antigen. Formulations of therapeutic and diagnostic agents may be prepared by mixing the antibody with the pharmaceutically acceptable carriers, excipients, or stabilizers in forms such as lyophilized powders, slurries, aqueous solutions, or suspensions. The term "pharmaceutically acceptable" refers to molecule, molecule fragment, or composition that does not produce unfavorable, allergic or other adverse effects when properly administered to animals or humans. Specific examples of some substances that may act as pharmaceutically acceptable carriers or components include sugars (such as lactose), starches, celluloses and their derivatives, vegetable oils, gelatins, polyols (such as propylene glycol), alginates, and the like.

The antibody of the present invention or the nucleic acids or polynucleotides encoding the antibody may be used alone or in combination with one or more other therapeutic agents, such as vaccines.

The term "pharmaceutically acceptable carrier and/or excipient and/or stabilizer" refers to a carrier and/or excipient and/or stabilizer that are pharmacologically and/or physiologically compatible with a subject and an active ingredient and that are non-toxic to cells or mammals exposed to the carrier and/or excipient and/or stabilizer at the dose and concentration employed. The pharmaceutically acceptable carrier and/or excipient and/or stabilizer include, but are not limited to, a PH adjuster, a surfactant, an adjuvant, an ionic strength enhancer, a diluent, a reagent for maintaining osmotic pressure, a reagent for delaying absorption and a preservative. For example, the pH adjuster includes, but is not limited to, a phosphate buffered saline. The surfactant includes, but is not limited to, cationic, anionic, or non-ionic surfactants, such as Tween-80. The ionic strength enhancer includes, but is not limited to, sodium chloride. The preservatives includes, but is not limited to, various antibacterial and antifungal agents, such as parabens, chlorobutanol, phenol, sorbic acid, and the like. The agent for maintaining osmotic pressure includes, but is not limited to, sugar, NaCl, and analogues thereof. The agent for delaying absorption includes, but is not limited to, monostearates and gelatin. The Diluent includes, but is not limited to, water, an aqueous buffer (such as buffered saline), an alcohol, and a polyol (such as glycerol). The preservative includes, but is not limited to, various antibacterial and antifungal agents, such as thimerosal, 2-phenoxyethanol, parabens, chlorobutanol, phenol, sorbic acid, and the like. The stabilizer has the meaning commonly understood by those skilled in the art, capable of stabilizing the desired activity of the active ingredient in the pharmaceutical, including but not limited to sodium glutamate, gelatin, SPGA, sugars (for example, sorbitol, mannitol, starch, sucrose, lactose, dextran, or glucose), amino acids (for example, glutamic acid, glycine), proteins (for example, dried whey, albumin, or casein) or the degradation products thereof (for example, lactalbumin hydrolysates), and the like.

As used herein, the term "effective amount" refers to an amount sufficient to obtain or at least partially obtain the desired effect. For example, a prophylactically effective amount refers to an amount sufficient to prevent, arrest or delay the onset of a disease; a therapeutically effective amount refers to an amount sufficient to cure or at least partially arrest the disease and complications thereof in a patient already suffering from the disease. Determining such effective amounts is entirely within the capability of those skilled in the art. For example, the amount effective for therapeutic use depends on the severity of the to-be-treated disease, the overall state of the patient's immune system, the general condition of the patient such as age, weight and sex, the mode of administration of the drug, and other treatments administered concurrently.

Embodiments of the present application are further described by the examples given below. It is to be understood by those skilled in the art that the following drawings and examples are illustrative of the present application and are not intended to further limit the present application.

### Description of the Drawings

Figure 1: Assay of Binding Affinity between Anti-human TFPI Antibody and Human TFPI Protein.
Figure 2: Assay of Restoration of FXa Activity by Anti-human TFPI Antibody.
Figure 3: Assay of Neutralization of TFPI by Anti-human TFPI Antibody.
Figure 4: Assay of Binding of Anti-human TFPI Antibody to HUVEC Cells.
Figure 5: Assay of Neutralization of TFPI on HUVEC Cells by Anti-human TFPI Antibody.

### Detailed Embodiments

The present invention is now described with reference to the following examples, which are intended to illustrate the invention (instead of limiting the present invention).

Unless otherwise specified, experimental methods in molecular biology and immunoassays used in the present invention are substantially performed with reference to J. Sambrook et al., Molecular Cloning: A Laboratory Manual, 2nd edition, Cold Spring Harbor Laboratory, 1989, and F. M. Ausubel et al., Short Protocols in Molecular Biology, 3rd edition, John Wiley & Sons, Inc., 1995; and the use of restriction enzymes is in accordance with conditions recommended by the product manufacturer. It is known to by those skilled in the art that the examples illustrate the present invention by way of example and are not intended to limit the scope of the present invention as claimed.

### Example 1: Preparation of Mouse Monoclonal Antibody Against Human TFPI

Human TFPI antigen (protein sequence: NCBI accession number NP_006278.1) was fully emulsified with complete Freund's adjuvant and used to immunize male Balb/C mice at a dose of 50 µg per mouse through a multi-point immunization method once every three weeks. 10 days after the third immunization, blood was collected from the tail vein, and the plasma anti-human TFPI antibody titer was tested by ELISA to monitor the degree of immune response in the mice. Then, 3 days before fusion, the mouse with the highest anti-human TFPI antibody titer was given a booster immunization.3 days later, the mice were sacrificed and their spleens were removed and fused with the mouse myeloma Sp2/0 cell line. A mixture of 2×10⁸ Sp2/0 cells and 2×10⁸ spleen cells was fused in a solution of 50% polyethylene glycol (molecular weight 1450) and 5% dimethyl sulfoxide (DMSO). The spleen cells were adjusted to 5×10⁵/mL with Iscove's medium (containing 10% fetal bovine serum, 100 U/mL penicillin, 100 µg/mL streptomycin, 0.1 mM hypoxanthine, 0.4 µM aminopterin, and 16 µg thymidine), and 0.3 mL was added to each well of a 96-well plate and incubated at 37°C in a 5% CO₂ incubator. After 10 days of culture, a high-throughput ELISA method was used to detect clones with high-affinity binding of antibodies to TFPI in the supernatants. The fusion cells in the wells containing the above-mentioned monoclonal antibodies were then subcloned to further screen and obtain hybridoma cell line #3-71.

The clone producing the specific antibody was cultured in RPMI 1640 medium supplemented with 10% fetal calf serum (FCS). When the cell density reached approximately 5×10⁵ cells/mL, the medium was replaced with serum-free medium. 2 to 4 days later, the cultured medium was centrifuged to collect the supernatant. A protein G column was used for antibody purification. The monoclonal antibody elution was dialyzed against 150 mM NaCl. The dialyzed solution was sterile-filtered through a 0.22 µm filter to obtain the purified mouse monoclonal antibody against human TFPI, mAb3-71, ready for testing.

### Example 2: Affinity Measurement and Kinetic Study of Mouse-Derived TFPI Antibody

The binding affinity constant of the purified mouse monoclonal antibody against human TFPI (mAb3-71) to human TFPI was determined using biolayer interferometry (BLI) with the ForteBio Octet RED&QK system from PALL Corporation. The concentration gradient for multi-channel parallel quantitative analysis was set to 3.125, 6.25, 12.5, 25, 50, and 100 nM, and human TFPI with a His tag at 10 µg/mL was coupled to Ni-NTA sensors. The affinity assay results are shown in Table 1, indicating that the mouse monoclonal antibody exhibits extremely high binding affinity to human TFPI, with a KD value in the order of 10⁻¹² M.

**Table 1. Affinity Assay Results of Mouse Monoclonal Antibody**

| Antibody | KD (M) | Ka (1/Ms) | Kd (1/s) |
|---|---|---|---|
| mAb3-71 | 4.539E-12 | 2.893E+05 | 1.313E-06 |

### Example 3: Humanization of Mouse-Derived Anti-Human TFPI Antibody

The mouse-derived antibody was humanized using the CDR grafting method. The basic principle of the CDR grafting is to graft the CDRs of a murine antibody onto a human antibody template and simultaneously introduce several or some crucial FR (framework region) residues of the murine antibody that stabilize the CDR conformation and that are important to antigen-antibody binding into the human antibody template (backmutations), so as to reduce the immunogenicity of the murine antibody and maintain the affinity of the murine antibody. Besides the above CDR grafting operation, the isoelectric point (PI), hydrophobic aggregation, post-translational modification (PTM) such as glycosylation, cleavage and isomerization sites and immunogenicity of the humanized antibody after the CDR grafting are further calculated, and amino acids causing issues in these four aspects are mutated to ensure that the humanized antibody could fully exert its pharmacodynamic effects during clinical use.

A specific flow of antibody humanization is described below. The IMGT human antibody germline database (http://www.imgt.org/3Dstructure-DB/cgi/DomainGapAlign.cgi) was searched to obtain a human antibody template with high similarity to the murine antibodies. The CDRs of the murine antibodies and the human antibody template were annotated with Discovery Studio to define the CDRs according to the Kabat or IMGT scheme. The 6 CDRs of the human antibody template were replaced with the 6 CDRs of the murine antibody, respectively. Each of the 6 grafted CDR regions may be an amino acid region defined by Kabat or IMGT. After the CDR grafting, FR region backmutations from the murine antibody to the humanization template were performed. Crucial FR region amino acids of the murine antibody that stabilize the CDR conformation of the antibody and that are important to antigen-antibody binding include four classes of amino acid residues: (1) amino acids buried below the antibody surface within 6 Å of the CDR regions; (2) amino acids exposed on the antibody surface within 6 Å of the CDR regions; (3) interface amino acids between light and heavy chain domains of the antibody; and (4) vernier zone residues that stabilize the CDR conformation of the antibody (Foote J and Winter G, 1992, J Mol Biol, 224: 487-499). The above four classes of crucial FR region residues of the murine antibody are determined by establishing a three-dimensional structural model of the murine antibody. As for the four classes of amino acids of the human template that are inconsistent with the sequences of the murine antibody, the three-dimensional structure was analyzed, amino acids important for maintaining the CDR conformation and the antigen-antibody binding were selected, and the grafting or substitution of the amino acids from the murine antibody to the human template was performed. Then, the isoelectric point, hydrophobic aggregation, post-translational modification and immunogenicity of the humanized antibody produced after the grafting of the four classes of amino acids were further calculated, and problematic amino acids were mutated to obtain the final humanized antibody sequence.

Based on the above method, a humanized antibody named AB8D was constructed using the CDRs of the mouse-derived antibody mAb3-71. The amino acid sequences of the CDR regions contained in the variable regions of the mouse and humanized antibodies are shown in Table 2, and the amino acid sequences of the heavy chain and light chain variable regions are shown in Table 3.

**Table 2. Sequences of CDR Regions for Exemplary Anti-TFPI Murine and Humanized Antibodies**

| **Antibody Number** | **CDR** | **Kabat** | **IMGT** |
|---|---|---|---|
| mAb3-71 | HCDR1 | SYWMH | GYTFTSYW |
| | | (SEQ ID NO: 1) | (SEQ ID NO: 7) |
| | HCDR2 | EIKPDNGRINYNEKFKT | IKPDNGRI |
| | | (SEQ ID NO: 2) | (SEQ ID NO: 8) |
| | HCDR3 | SSVVDVAMDY | ARSSVVDVAMDY |
| | | (SEQ ID NO: 3) | (SEQ ID NO: 9) |
| | LCDR1 | SASSSVSSSYLY | SSVSSSY |
| | | (SEQ ID NO: 4) | (SEQ ID NO: 10) |
| | LCDR2 | GTSILAS | GTS |
| | | (SEQ ID NO: 5) | (SEQ ID NO: 11) |
| | LCDR3 | HQWSHYPFT | HQWSHYPFT |
| | | (SEQ ID NO: 6) | (SEQ ID NO: 6) |
| AB8D | HCDR1 | SYWMH | GYTFTSYW |
| | | (SEQ ID NO: 1) | (SEQ ID NO: 7) |
| | HCDR2 | EIKPDQGRINYNEKFKT | IKPDQGRI |
| | | (SEQ ID NO: 12) | (SEQ ID NO: 13) |
| | HCDR3 | SSVVDVAMDY | ARSSVVDVAMDY |
| | | (SEQ ID NO: 3) | (SEQ ID NO: 9) |
| | LCDR1 | SASSSVSSSYLY | SSVSSSY |
| | | (SEQ ID NO: 4) | (SEQ ID NO: 10) |
| | LCDR2 | GTSILAS | GTS |
| | | (SEQ ID NO: 5) | (SEQ ID NO: 11) |
| | LCDR3 | HQWSHYPFT | HQWSHYPFT |
| | | (SEQ ID NO: 6) | (SEQ ID NO: 6) |

**Table 3. Amino Acid Sequences of Murine and Humanized Antibody Variable Regions**

| | VH Amino Acid Sequence | VL Amino Acid Sequence |
|---|---|---|
| mAb3-71 | SEQ ID NO: 14 | SEQ ID NO: 15 |
| AB8D | SEQ ID NO: 16 | SEQ ID NO: 17 |

To obtain the full-length antibody sequence consisting of two heavy chains and two light chains, the VH and VL sequences shown in Table 3 can be spliced or assembled with the sequences of the antibody heavy chain constant region (preferably derived from human IgG1, IgG2, or IgG4) and the light chain constant region (preferably derived from human κ light chain, with the amino acid sequence as shown in SEQ ID NO: 18) using conventional techniques. Preferably, the heavy chain constant region is the human wild-type heavy chain constant region or its mutant.

In an exemplary embodiment, the humanized antibody molecule includes the heavy chain constant region of wild-type human IgG1 (with the amino acid sequence as shown in SEQ ID NO: 19). In another embodiment, the humanized antibody molecule includes the heavy chain constant region of human IgG1 mutated at M252Y, S254T, T256E, and M428L according to EU numbering (with the amino acid sequence as shown in SEQ ID NO: 20). In another embodiment, the humanized antibody molecule includes the heavy chain constant region of wild-type human IgG2 (with the amino acid sequence as shown in SEQ ID NO: 21). Alternatively, a modified human IgG4 constant region sequence is used. In one embodiment, the humanized antibody molecule includes human IgG4 mutated at position 228 (such as S to P) according to EU numbering (with the amino acid sequence as shown in SEQ ID NO: 22).

In an exemplary embodiment, the heavy chain amino acid sequence of the humanized antibody molecule is as shown in SEQ ID NO: 23, and the light chain amino acid sequence is as shown in SEQ ID NO: 24.

### Example 4: Construction, Expression, and Preparation of Anti-Human TFPI Antibody Expression Vectors

Based on the heavy chain and light chain sequences obtained from the previous examples, cDNAs encoding these sequences were designed and inserted into the pcDNA3.1 eukaryotic expression vector to construct a humanized antibody expression vector. This expression vector plasmid contains the cytomegalovirus early gene promoter-enhancer required for high-level expression in mammalian cells. Additionally, the plasmid contains a selectable marker gene that confers ampicillin resistance in bacteria and G418 resistance in mammalian cells. Furthermore, the plasmid incorporates the dihydrofolate reductase (DHFR) gene, which in appropriate host cells, the antibody gene and the DHFR gene can be co-amplified with methotrexate (MTX).

The recombinant expression vector plasmid constructed above was transfected into a mammalian host cell line to express the humanized antibody. For stable high-level expression, the preferred host cell line is DHFR-defective Chinese hamster ovary (CHO) cells (see U.S. Patent No. 4,818,679). The preferred transfection method is electroporation, and other methods including calcium phosphate co-precipitation, lipofection and protoplasmic fusion may also be used. In electroporation, a GenePulser (Bio-Rad Laboratories) set at 300 V and 1050 µFd capacitance was used, with 2 × 10⁷ cells suspended in 0.8 mL of PBS containing 20 µg of the expression vector plasmid added to the cuvette. Two days post-transfection, 0.2 mg/mL G418 and 200 nM MTX (Sigma) were added. To achieve a relatively high level of expression, the transfected antibody gene was co-amplified with the MTX-inhibited DHFR gene.Transfectants were subcloned by limiting dilution and the secretion rate of each cell line was determined by the ELISA, and cell clones with high-level antibody expression were selected. Conditioned media containing the antibodies were collected for assessment of their biological activities *in vitro* and *in vivo.*

### Example 5: Purification and Characterization of Anti-Human TFPI Antibody

This example illustrates the purification and characterization methods for the humanized antibody AB8D. The cell culture supernatant was clarified by high-speed low-temperature centrifugation or deep filtration, 0.22 µ m sterilization filtration, and further purified by three-step chromatography including protein A affinity, anion exchange, and cation exchange. The specific methods are as follows:
In the first step, Protein A affinity chromatography is used for capture. The equilibration buffer is PBS, and linear elution is performed using an elution buffer (50 mM NaAc-HAc, pH 3.7). The pH of the Protein A elution product is adjusted to 3.6-3.8 with 1M glacial acetic acid, and the product is incubated at room temperature for 60 minutes for virus inactivation. Then, 2M Tris is added to neutralize the pH to 5.5.

For intermediate purification, the anion exchange resin Q Bestarose FF is selected to remove residual DNA, endotoxins, impurity proteins, etc. The equilibration buffer is 50 mM NaAc-HAc, pH 5.5. Proteins that flow through the anion exchange medium are directly bound to the cation exchange medium MonomixHC45-SP. The cation exchange chromatography equilibration buffer is 50 mM NaAc-HAc, pH 5.5, and linear elution is performed using an elution buffer (50 mM NaAc-HAc, 0.2 M NaCl, pH 5.5).

The protein solution eluted from the cation exchange medium is then filtered through a nanofiltration membrane, concentrated by ultrafiltration, and sterile-filtered to obtain the bulk solution.

Conduct SEC-HPLC purity analysis and SDS-PAGE electrophoresis analysis on the stock solution. The SEC-HPLC results show that the purity of the main peak of the purified antibody is over 99%. The theoretical molecular weight of the AB8D antibody is approximately 145 KD. Under reducing conditions, SDS-PAGE electrophoresis shows that the light chain is approximately 25 KD and the heavy chain is approximately 50 KD, which is basically consistent with the theoretical values.

### Example 6: Evaluation of In Vitro Biological Functions of Anti-Human TFPI Antibody

### 6.1 Determination of the Affinity of Anti-Human TFPI Antibody to Human TFPI Protein

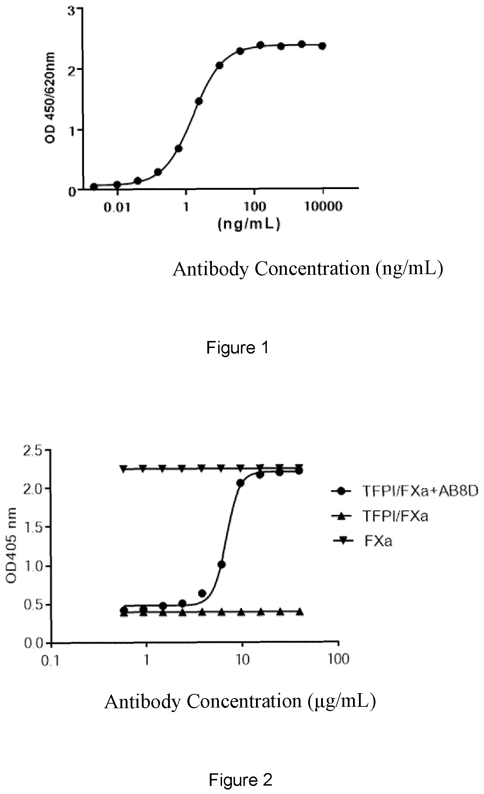

Human TFPI was coated onto the ELISA plate at a concentration of 0.1 µg/mL, with 100 µL per well, and incubated overnight at 4°C. The coating solution was then discarded, and the wells were blocked with 200 µL of PBS solution containing 1% BSA (PBSB) per well at 37°C for 1 hour. The plate was washed with PBS solution containing 0.05% Tween-20 (PBST). A series of diluted concentrations of AB8D were added, with 100 µL per well, and incubated for 1 hour at 37°C. The plates were washed with PBST, and then HRP-labeled goat anti-human IgG (H+L) (Jackson Laboratory) was added as the detection antibody, with incubation for 1 hour at 37°C. After washing three times with 0.05% PBST, TMB was added, with 100 µL per well, and the color was developed at room temperature for 5 minutes. The reaction was terminated by adding 0.2 M H₂SO₄, with 50 µL per well. The absorbance values were read at dual wavelengths of 450/620 nm using a microplate reader. Using OD450 nm - OD620 nm as the Y-axis and antibody concentration as the X-axis, the data were analyzed using GraphPad Prism 6 software with a four-parameter logistic curve fitting to calculate the EC₅₀ value. As shown in Figure 1, AB8D exhibited affinity for human TFPI protein with an EC₅₀ of 1.65 ng/mL.

### 6.2 FXa Activity Assay

Human TFPI was diluted in Buffer 1 (containing 20 mM HEPES, 150 mM NaCl, 5 mM CaCl₂, 0.5 mg/ml BSA, pH 8.0) to a concentration of 10 µg/mL and added to a 96-well flat-bottom plate at 50 µL per well. A series of diluted concentrations of AB8D were then added, with 50 µL per well, and incubated at 37°C for 30 minutes. FXa (purchased from Haematologic Technologies) was diluted in Buffer 1 to a concentration of 1 µg/mL and added at 50 µL per well, followed by incubation at 37°C for 30 minutes. The substrate S-2765 (purchased from CHROMOGENIX) was diluted in Buffer 1 to 2 mM and added at 50 µL per well. After the reaction, the absorbance values at 405 nm were read using a microplate reader. The data were plotted using GraphPad Prism 6 software, with OD405 nm as the Y-axis and antibody concentration as the X-axis. As shown in Figure 2, AB8D was able to restore the activity of FXa inhibited by TFPI, with an EC₅₀ of 6.9 µg/mL.

### 6.3 TFPI Neutralization Test: Measurement of TF/FVIIa/FXa Inhibition Effect

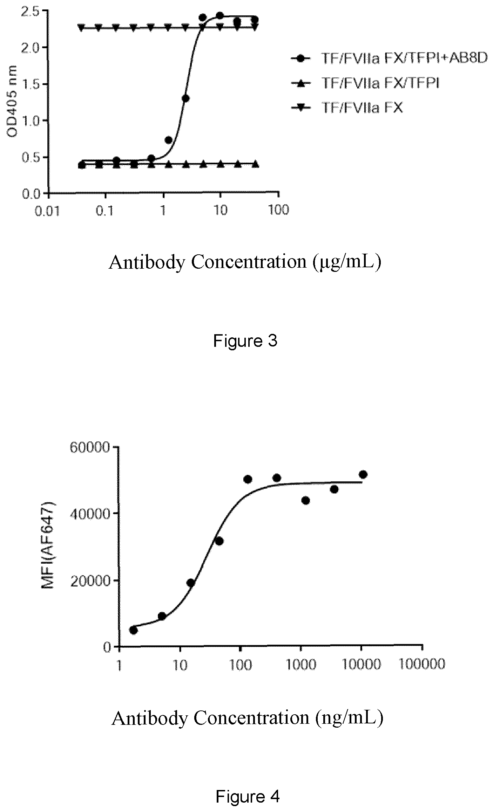

TF (Tissue Factor, purchased from Beijing Borxi Technology Co., Ltd) and FVIIa (WHO) were diluted in Buffer 1 to concentrations of 1.4 ng/mL for TF and 45 IU/mL for FVIIa, respectively. Then, 25 µL of each was added to a 96-well plate and incubated at 37°C for 10 minutes. A series of diluted concentrations of AB8D was then added to the plate, with 25 µL per well. Human TFPI was diluted in Buffer 1 to a concentration of 10 µg/mL and added at 25 µL per well. The plate was then incubated at 37°C for 30 minutes. FX (purchased from Haematologic Technologies) was diluted in Buffer 1 to a concentration of 44.2 µg/mL and added at 25 µL per well, followed by incubation at 37°C for 30 minutes. The reaction was terminated by adding 75 mM EDTA, with 10 µL per well. The substrate S-2765 was diluted in Buffer 1 to 2 mM and added at 50 µL per well. After the reaction, the absorbance values at 405 nm were read using a microplate reader. The data were analyzed using GraphPad Prism 6 software, with OD405 nm as the Y-axis and antibody concentration as the X-axis. A four-parameter logistic curve fitting was performed to calculate the EC₅₀ value. As shown in Figure 3, at the molecular level, AB8D can neutralize the inhibitory effect of TFPI, restoring the activity of FXa inhibited by TFPI-mediated FVIIa/TF in a dose-dependent manner, with an EC₅₀ of 3.17 µg/mL.

### 6.4 Binding Detection of AB8D to TFPI Expressed on HUVECs by FACS

HUVEC cells (Human Umbilical Vein Endothelial Cell Line, purchased from Shanghai Biological Technology Co., Ltd.) were cultured and collected by centrifugation. The cells were resuspended in 1% PBSB and adjusted to a density of 1×10⁶ cells/mL. Then, 100 µL of the cell suspension was added to each well of a 96-well U-bottom plate and blocked at 4°C for 30 minutes. The cells were washed once with 1% PBSB. A series of diluted concentrations of AB8D was then added to the wells, with 100 µL per well, and incubated at 4°C for 1 hour. The supernatants were removed by centrifugation, and the cells were washed twice with 1% PBSB. Diluted AF647 goat anti-human IgG (H+L) antibody (Jackson Immuno Research Inc., diluted 1:400) was added to each well at 100 µL, and incubated at 4°C in the dark for 1 hour. The supernatants were again removed by centrifugation, and the plate was washed twice with 1% PBSB. The cells were resuspended in 1% PBSB at 150 µL per well, and the signal intensity was detected using a flow cytometer. The data were analyzed using GraphPad Prism 6 software, with mean fluorescence intensity as the Y-axis and antibody concentration as the X-axis. A four-parameter logistic curve fitting was performed to calculate the EC₅₀ value. As shown in Figure 4, AB8D can bind to HUVECs through TFPI, with an EC₅₀ of 28.6 ng/mL.

### 6.5 Neutralization Effect of Anti-Human TFPI Antibody on TFPI in HUVECs

When the fusion rate of HUVEC cells reaches over 80% and the appearance is good, the culture medium was discarded, and the culture dish was rinsed with sterile phosphate-buffered saline (PBS). Add 1 mL of cell digestion solution to each culture dish and incubate at room temperature for 1 minute. Add 2mL ECM medium containing 10% fetal bovine serum to each culture dish to terminate cell digestion and resuspend the cells. The cell suspension was transferred to a 15-mL centrifuge tube and centrifuged at 800 rpm for 5 minutes. The supernatant was discarded, and an appropriate amount of medium was added to resuspend the cells, which were gently mixed by pipetting. Cell counting was performed, and the cell density was adjusted to 5×10⁵ cells/mL. The cells were gently mixed and plated in a 96-well plate at 100 µL per well. The plate was then placed in a CO₂ incubator for overnight culture.

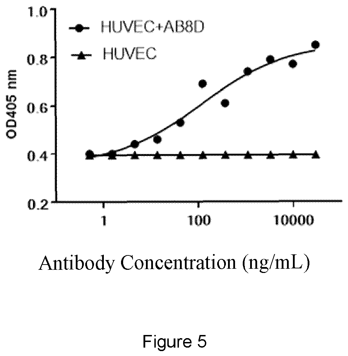

The next day, the cell culture medium was discarded, and the cells were washed twice with DPBS. A mixture of TNFα (400 ng/mL) and IL-1β (400 ng/mL) was prepared and added to each well at 25 µL. Then, a series of diluted concentrations of AB8D in Buffer 2 (containing 25 mM HEPES, 137 mM NaCl, 3.5 mM KCl, pH 7.4) was added to the wells at 25 µL per well. The plate was placed in a CO₂ incubator for 2 hours, with Buffer 2 used as the blank control. FVIIa was diluted in Buffer 3 (containing 25 mM HEPES, 137 mM NaCl, 3.5 mM KCl, 5 mM CaCl₂, 1 mg/ml BSA, pH 7.4) to a concentration of 45 IU/mL and added to each well at 25 µL, followed by incubation at 37°C for 15 minutes. FX was diluted in Buffer 3 to a concentration of 400 nM and added to each well at 25 µL, followed by incubation at 37°C for 40 minutes. 40µL of supernatant was taken and transferred to a new 96-well plate, and 75 mM EDTA was added to each well at 10 µL to terminate the reaction. The substrate S-2765 was diluted in Buffer 3 to 1.2 mM and added to each well at 50 µL. After the reaction, the absorbance values at 405 nm were read using a microplate reader. The data were plotted using GraphPad Prism 6 software, with OD405 nm as the Y-axis and antibody concentration as the X-axis. A four-parameter logistic curve fitting was performed to calculate the EC₅₀ value. As shown in Figure 5, AB8D can neutralize TFPI in HUVECs, blocking TFPI-mediated inhibition of FVIIa/TF and restoring FXa activity in cellular-level, with an EC₅₀ of 117.3 ng/mL.

### Example 7: In Vivo Pharmacodynamic Evaluation of Anti-Human TFPI Antibody

24 male New Zealand rabbits (2.0 to 2.5 kg) purchased from Shanghai Jiesijie Laboratory Animal Co., Ltd. were randomly divided into 8 groups based on their body weight: G1 (vehicle control group), G2 (AB8D-10 mg/kg group), G3 (AB8D-5 mg/kg group), G4 (AB8D-2.5 mg/kg group), G5 (vehicle control group), G6 (AB8D-20 mg/kg group), G7 (AB8D-10 mg/kg group), and G8 (AB8D-5 mg/kg group).

The corresponding vehicle control or different concentrations of AB8D test articles were administered subcutaneously to each group. On the 4th and 7th day after administration, the G1-G4 and G5-G8 groups were given a single injection of 15mg/kg of anticoagulant factor VIII neutralizing antibody (anti-FVIII neutralizing antibody) via the marginal ear vein of rabbits to establish a hemophilia model. 45 minutes after the administration of the anti-FVIII neutralizing antibody in each group, the rabbits were anesthetized with 2%-5% isoflurane, the anesthetized rabbits were placed on a prone position, and one of their front paws was pre-immersed in a beaker of 37°C saline. The third toenail was cut to induce cuticle bleeding. After cutting, the paw was allowed to hang naturally into the beaker of 37°C saline, with the front paw submerged below the surface of the saline. The bleeding time was recorded, and the blood loss was measured using a gravimetric method simultaneously. If the bleeding time exceeded 60 minutes, it was recorded as 60 minutes.

As shown in Table 4 and table 5, AB8D at doses ≥ 5mg/kg had an inhibitory effect on both the amount and time of bleeding in the stratum corneum of hemophilia model rabbits after a single administration of AB8D on day 4 and day 7.

**Table 4. Bleeding Volume and Bleeding Time after a Single Dose Administration on the Day 4 (mean±SEM, n=3)**

| Group | Bleeding Volume (g) | Bleeding Duration (seconds) |
|---|---|---|
| | Day 4 | Day 4 |
| Group G1 | 13.96±2.08 | 1782.00±913.45 |
| Group G2 | 6.26±0.43 | 711.33±2.40 |
| Group G3 | 4.10±0.53 | 699.67±11.46 |
| Group G4 | 19.45±4.15 | 1447.33±507.33 |

**Table 5. Bleeding Volume and Bleeding Time after a Single Dose on the Day 7 (mean±SEM, n=3)**

| Group | Bleeding Volume (g) | Bleeding Duration (seconds) |
|---|---|---|
| | Day 7 | Day 7 |
| Group G5 | 46.85±10.44 | 3600.00±0.00 |
| Group G6 | 13.65±0.90 | 2774.33±825.67 |
| Group G7 | 9.05±1.86 | 1804.33±345.28 |
| Group G8 | 13.68±2.91 | 1944.00±338.96 |

Though the preferred examples of the present invention are illustrated and described, it is to be understood that those skilled in the art may make various changes in accordance with the teachings herein within the scope of the present invention.

All the publications mentioned in the present invention are incorporated herein by reference as if each publication is separately incorporated herein by reference. In addition, it is to be understood that those skilled in the art, who have read the preceding content of the present invention, may make various changes or modifications to the present invention, and these equivalent forms also fall within the scope of the appended claims.

## Claims

1. An antibody or an antigen-binding fragment thereof capable of specifically binding to TFPI, wherein the heavy chain variable region (VH) of the antibody or the antigen-binding fragment thereof comprises at least one, two, or three complementarity-determining regions (CDRs) selected from the group consisting of:
(1) HCDR1 having a sequence as shown in SEQ ID NO: 1 or 7, or a sequence having one or several amino acid substitutions, deletions, or additions (such as 1, 2, or 3 substitutions, deletions, or additions) relative to any of the sequences;
(2) HCDR2 having a sequence as shown in SEQ ID NO: 2, 8, 12, or 13, or a sequence having one or several amino acid substitutions, deletions, or additions (such as 1, 2, or 3 substitutions, deletions, or additions) relative to any of the sequences; and
(3) HCDR3 having a sequence as shown in SEQ ID NO: 3 or 9, or a sequence having one or several amino acid substitutions, deletions, or additions (such as 1, 2, or 3 substitutions, deletions, or additions) relative to any of the sequences;
and/or, the light chain variable region (VL) of the antibody or antigen-binding fragment thereof comprises at least one, two, or three CDRs selected from the group consisting of:
(4) LCDR1 having a sequence as shown in SEQ ID NO: 4 or 10, or a sequence having one or several amino acid substitutions, deletions, or additions (such as 1, 2, or 3 substitutions, deletions, or additions) relative to any of the sequences;
(5) LCDR2 having a sequence as shown in SEQ ID NO: 5 or an amino acid sequence of Gly-Thr-Ser, or a sequence having one or several amino acid substitutions, deletions, or additions (such as 1, 2, or 3 substitutions, deletions, or additions) relative to any of the sequences; and
(6) LCDR3 having a sequence as shown in SEQ ID NO: 6, or a sequence having one or several amino acid substitutions, deletions, or additions (such as 1, 2, or 3 substitutions, deletions, or additions) relative to any of the sequences;
wherein the CDRs contained in the heavy chain variable region and/or the CDRs contained in the light chain variable region are defined by the Kabat or IMGT numbering system;
preferably, the substitutions mentioned in any one of (1)-(6) are conservative substitutions.

2. The antibody or the antigen-binding fragment thereof according to claim 1, wherein the antibody or the antigen-binding fragment thereof comprises three VH variable region CDRs and three VL variable region CDRs selected from the following group:
(1) HCDR1, HCDR2, HCDR3, LCDR1, LCDR2, and LCDR3 have sequences as shown in SEQ ID NOs: 1, 2, 3, 4, 5, or 6, respectively, or sequences having one or several amino acid substitutions, deletions, or additions (such as 1, 2, or 3 substitutions, deletions, or additions) compared to any of the aforementioned sequences;
(2) HCDR1, HCDR2, HCDR3, LCDR1, LCDR2, and LCDR3 have sequences as shown in SEQ ID NOs: 7, 8, 9, 10, Gly-Thr-Ser, or 6, respectively, or sequences having one or several amino acid substitutions, deletions, or additions (such as 1, 2, or 3 substitutions, deletions, or additions) relative to any of the sequences;
(3) HCDR1, HCDR2, HCDR3, LCDR1, LCDR2, and LCDR3 have sequences as shown in SEQ ID NOs: 1, 12, 3, 4, 5, or 6, respectively, or sequences having one or several amino acid substitutions, deletions, or additions (such as 1, 2, or 3 substitutions, deletions, or additions) relative to any of the sequences;
(4) HCDR1, HCDR2, HCDR3, LCDR1, LCDR2, and LCDR3 have sequences as shown in SEQ ID NOs: 7, 13, 9, 10, Gly-Thr-Ser, or 6, respectively, or sequences having one or several amino acid substitutions, deletions, or additions (such as 1, 2, or 3 substitutions, deletions, or additions) relative to any of the sequences.

3. The antibody or the antigen-binding fragment thereof according to claim 2, wherein the antibody or the antigen-binding fragment thereof is murine-derived or chimeric, wherein its heavy chain variable region comprises a heavy chain FR region of murine IgG1, IgG2, IgG3, or a variant thereof; and its light chain variable region comprises a light chain FR region of murine κ, λ chain, or a variant thereof.

4. The antibody or the antigen-binding fragment thereof according to claim 3, wherein the antibody or the antigen-binding fragment thereof comprises the following VH and VL sequences: the VH domain comprises an amino acid sequence as shown in SEQ ID NO: 14, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 14; and the VL domain comprises an amino acid sequence as shown in SEQ ID NO: 15, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 15.

5. The antibody or the antigen-binding fragment thereof according to claim 2, wherein the antibody or antigen-binding fragment is humanized.

6. The antibody or the antigen-binding fragment thereof according to claim 5, wherein the antibody or the antigen-binding fragment thereof comprises the following VH and VL sequences: the VH domain comprises an amino acid sequence as shown in SEQ ID NO: 16, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 16; and the VL domain comprises an amino acid sequence as shown in SEQ ID NO: 17, or having a sequence that is substantially identical to (for example, having at least 80%, 85%, 90%, 92%, 95%, 97%, 98%, 99% or higher identity or having one or more amino acid substitutions (such as conservative substitutions) relative to) SEQ ID NO: 17.

7. The antibody according to claim 5, wherein the antibody comprises a heavy chain constant region and a light chain constant region derived from human immunoglobulins; preferably, the heavy chain constant region is selected from the heavy chain constant regions of human IgG1, IgG2, IgG3, and IgG4; and the heavy chain constant region has a native sequence or a sequence with one or more amino acid substitutions, deletions, or additions relative to the native sequence from which it is derived; and the light chain constant region is preferably the constant region of human κappa chain as shown in SEQ ID NO: 18.

8. The antibody according to claim 7, wherein the heavy chain constant region contained in the antibody is selected from the following group:
(i) a heavy chain constant region of wild-type human IgG1, as shown in SEQ ID NO: 19;
(ii) a heavy chain constant region of human IgG1 containing the M252Y, S254T, T256E, and M428L mutations, as shown in SEQ ID NO: 20;
(iii) a heavy chain constant region of wild-type human IgG2, as shown in SEQ ID NO: 21;
(iv) a heavy chain constant region of human IgG4 containing an S228P mutation, as shown in SEQ ID NO: 22.

9. The antibody according to claim 7, wherein the antibody has a heavy chain with an amino acid sequence as shown in SEQ ID NO: 23 and a light chain with an amino acid sequence as shown in SEQ ID NO: 24.

10. The antibody or the antigen-binding fragment thereof according to any one of claims 1-9, wherein the antibody or the antigen-binding fragment thereof is capable of binding to TFPI with a KD of 10 nM or lower.

11. A DNA molecule encoding the antibody or the antigen-binding fragment thereof according to any one of claims 1-9.

12. A vector containing the DNA molecule according to claim 11.

13. A host cell containing the vector according to claim 12, wherein the host cell comprises a prokaryotic cell, a yeast cell or a mammalian cell, preferably a CHO cell.

14. A pharmaceutical composition comprising an antibody or the antigen-binding fragment thereof according to any one of claims 1-9, and a pharmaceutically acceptable excipient, carrier, or diluent.

15. A method for preparing an antibody or the antigen-binding fragment thereof according to any one of claims 1-9, comprising: (a) obtaining a gene encoding the antibody or the antigen-binding fragment thereof and constructing an expression vector for the antibody or the antigen-binding fragment thereof; (b) transfecting the expression vector into host cells using genetic engineering methods; (c) culturing the host cells under conditions that allows the antibody or the antigen-binding fragment thereof to be produced; (d) isolating and purifying the produced antibody or antigen-binding fragment thereof;
wherein, in step (a), the expression vector is selected from one or more of plasmids, bacteria, and viruses, preferably a pcDNA3.1 vector;
wherein, in step (b), the constructed vector is transfected into the host cells using genetic engineering methods, and the host cells include aprokaryotic cells, yeast cells, or mammalian cells, preferably CHO cells;
wherein, in step (d), the antibody or the antigen-binding fragment thereof is isolated and purified using conventional immunoglobulin purification methods, including Protein A affinity chromatography and ion exchange methods.

16. Use of the antibody or the antigen-binding fragment thereof according to any one of claims 1-9 for preparing a medicament, wherein the medicament is used for prevention or treatment of diseases or events associated with inherited or acquired coagulation factor deficiencies.

17. Use of the antibody or the antigen-binding fragment thereof according to any one of claims 1-9 for preparing a medicament, wherein the medicament is used for prevention or treatment of diseases or events associated with inherited or acquired coagulation factor deficiencies, wherein the disease is hemophilia.
